# EUROPÄISCHE PATENTANMELDUNG

(11) **EP 1 114 639 A2**
(43) Veröffentlichungstag der Anmeldung: **11.07.2001**
(21) Anmeldenummer: 00126458.9
(22) Anmeldetag: 07.12.2000
(51) Int. Cl.: A61K 7/50

(54) **Verwendung eines Co-Tenside im Gemisch mit einem Tenside (z.B. Natrium LES) zur Herstellung besonders hautverträglicher kosmetischer oder dermatologischer Reinigungszubereitungen**

(30) Priorität: 16.12.1999 DE 19960767
(71) Anmelder: Beiersdorf Aktiengesellschaft, 20245 Hamburg (DE)
(72) Erfinder: Schmucker, Robert, Dr., 20257 Hamburg (DE)

(57) **Zusammenfassung**

Verwendung eines oder mehrerer Co-Tenside (Tensid B) im Gemisch mit einem oder mehreren Tensiden die sich von Tensid B unterscheiden (Tensid A), zur Verminderung der Bindung von Tensid A an die Hautoberfläche.

## Beschreibung

Die vorliegende Erfindung betrifft ein Verfahren zur Herstellung besonders hautverträglicher kosmetischer oder dermatologischer Reinigungszubereitungen.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Die vorliegende Erfindung betrifft in einer besonderen Ausführungsform Reinigungszubereitungen für die Verwendung als Duschpräparat, Badepräparat, Körper- und Gesichtsreinigung

Auch derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab-bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze noch verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Aufgabe der vorliegenden Erfindung war somit, diesem Mangel des Standes der Technik Abhilfe zu schaffen. Weiterhin war eine Aufgabe der Erfindung, Wannen- aber auch Duschbadzubereitungen zur Verfügung zu stellen, welche einesteils hohe Pflegewirkung besitzen, ohne daß andererseits die reinigende Wirkung dahinter zurücksteht.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren oder solubilisieren können

Diese Stoffe schwemmen nicht nur Schmutz von Haut und Haaren, sie reizen, je nach Wahl des Tensids oder des Tensidgemisches, Haut und Schleimhäute mehr oder minder stark.

Eines der weltweit am häufigsten eingesetzten Tenside für kosmetische Zusammensetzungen ist das Natriumlaurylethersulfat. An sich ein ausgezeichnetes waschaktives Agens mit gutem Schaumbildungsvermögen wirkt es in höheren Dosen jedoch auf Haut und Schleimhäute reizend.

Wie jüngere Untersuchungen zeigen, wird das Reizpotential von Natriumlaurylethersulfat wenigstens teilweise dadurch gefördert, daß diese Substanz an die Hautoberfläche bindet und dort ein gewisses Reservoir bildet. Studien legen nahe, daß aus diesem Reservoir eine Penetration des Laurylethersulfates in tiefere Hautschichten erfolgt, wo es dann unkontrollierte Folgereaktionen eingehen kann, die ein erhöhtes Reizrisko bergen.

Das handelsübliche Natriumlaurylethersulfat (= Natriumpolyoxyethylenlaurylsulfat, nach INCI-Nomenklatur: "Sodium laureth sulfate"; CAS-Nr.1335-72-4) stellt, wie die meisten kosmetisch angewandten Rohstoffe keinen reinen Stoff dar, sondern - herstellungsbedingt - vielmehr ein Gemisch aus Substanzen, deren Strukturen der allgemeinen Formel 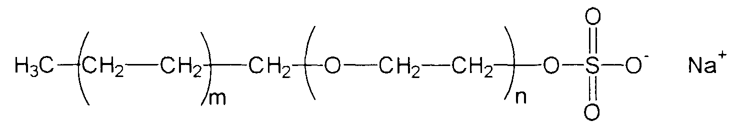 gehorchen, wobei n Zahlen von 0 bis 10 und m Zahlen von 4 bis 6 annehmen. Das in den Handelsprodukten hauptsächlich vertretene, namensgebende Lauryletherderivat hat m = 5, n=2-3. Handelsprodukte sind beispielsweise Texapon® N 25, Texapon® N 40, Texapon® N 70 und Texapon® N 103 der Gesellschaft Henkel KGaA.

Es existieren allerdings auch andere Laurylethersulfate, welche als Gegenion beispielsweise mit Alkylgruppen oder Hydroxyalkylgruppen substituierte oder auch unsubstituierte Ammoniumionen aufweisen, aber auch Magnesium und dergleichen mehr.

Wegen der guten Verfügbarkeit, des akzeptablen Preises und der ausgezeichneten Wascheigenschaften des Natriumlaurylethersulfates ist es allerdings in der Praxis nicht möglich, in absehbarer Zukunft auf diesen Stoff ganz zu verzichten. Zwar sind laurylethersulfatfreie Zubereitungen bekannt und durchaus vorteilhaft, sie zeichnen sich jedoch durch andere anwendungs- oder herstellungstechnische oder wirtschaftliche Nachteile aus.

Durch Langzeitapplikation (beispielsweise länger als 1 Stunde) oder wiederholte Kurzzeitapplikation von anionischen Tensiden kann es zu einer Herabsetzung der Hautfeuchtigkeit oder einer Erhöhung des Transepidermalen Wasserverlusts (TEWL) kommen.

Es ist an sich bekannt, Natriumlaurylethersulfat in Kombination mit anderen Tensiden als waschaktives Agens zu verwenden. Der Fachmann, welcher die Hautverträglichkeit solcher Zubereitungen steigern möchte, ersetzt dann einen Teil des Natriumlaurylethersulfates durch mildere Tenside. In der Regel sind aber als unerwünschte Nebeneffekte eine Verminderung der Schaumbildung und/oder der Reinigungsleistung in Kauf zu nehmen. Diesem Übelstande galt es also, Abhilfe zu schaffen.

Bei dem Versuch, auf der Basis von naturwissenschaftlichen Gesetzmäßigkeiten die Verträglichkeit von Laurylethersulfat zu erhöhen, ohne das Tensid durch andere, besser verträgliche Tenside (= Cotenside) zu ersetzen, stieß der Fachmann auf folgenden - bisher ungelösten - Widerspruch:
- Die Hautverträglichkeit von Tensiden korreliert mit der Monomerenkonzentration/CMC der Tenside. Diese nimmt bei Konzentrationen oberhalb der CMC nicht weiter zu
   *Imokawa G, Mishima Y. Contact Dermatitis. 1979: 5: 357*
   *Breuer MM. J Soc Cosmet Chem. 1979: 30: 41*
- Der Grad der Hautschädigung steigt mit steigender Tensidkonzentration - auch oberhalb der CMC.
   *Wilhelm KP, Surber C, Maibach Hl. Arch Dermatol Res. 1989: 281: 293-295.*

Aufgrund dieses Widerspruchs war für den Entwickler nicht klar, nach welchen Gesetzmäßigkeiten ein gegebenes Tensidsystem - in diesem Falle Laurylethersulfat - milder formuliert werden kann.

In verschiedenen Veröffentlichungen wurde spekuliert, daß zwischen der Hautreaktion und der Adsorption von Tensiden an der Haut ein Zusammenhang besteht. Sämtliche Untersuchungen zur Adsorption von Tensiden an Haut wurden jedoch entweder
- in vitro an Substraten mit begrenzter Ähnlichkeit zur menschlichen Haut (Kalluspulver, Hautpulver, isoliertes Human- oder Säugetier-Stratum corneum)
   *Dominguez JG, Parra JL, Infante MR, Pelejero CM, Balaguer F, Sastre T J Soc Cosmet Chem. 1977: 28: 165*
   *Garret HE. Trans of St. John's Hosp Dermatol Soc. 1965: 51: 166*
   *Faucher JA, Goddard ED. J Soc Cosmet Chem. 1978: 29: 323*
   *Gibson WT, Teall MR. Fd Chem Toxic. 1982: 21 581*
- ex vivo (exzidierte Human- oder Säugetierhaut)
   *Fullerton A, Broby-Johansen U, Agner T. Contact Dermatitis. 1994: 30: 222* oder
- in vivo mit unzulänglichen, irrelevanten, weil anwendungsfernen, oder nicht validierten Methoden (indirekte Färbemethoden, Extraktion mit Wasser oder Aceton)
   Imokawa G, Mishima Y. Contact Dermatitis. 1979: 5: 357 durchgeführt.

Unter Gleichgewichtsbedingungen (Langzeitapplikation) durchgeführte Adsorptionsmessungen haben bei Anwendungsbedingungen (Duschen) keine Relevanz. In der Literatur beschriebene Messungen spiegeln die Anwendungssituation nicht hinreichend wieder und lieferten daher irrelevante Ergebnisse.

Auf der 2^{nd} Scientific Conference of the Asian Societies of Cosmetic Scientists, 1995, in Seoul, wurde ein Vortrag mit dem Titel "Development of High-Safety Facial Cleansers through Reduction of Cutaneous Surfactant Adsorption" gehalten, in welche, ein der vorliegenden Erfindung verwandtes Thema behandelt wurde.

Bei den vorgestellten "Facial Cleansers" handelt es sich jedoch um Seifenprodukte, welche jedoch nicht Inhalt unseres Patents sein sollen. Bei Seifen handelt es sich zwar ebenfalls um anionische Tenside, welche allerdings nur bei hohen pH-Werten ihre tensidische Wirkung entfalten können. Bei Hautkontakt können Seifen durch die Pufferwirkung der Haut ihre Basizität und hierdurch ihren Seifencharakter verlieren. Diese Eigenheit besitzen die von uns fokussierten synthetischen Detergentien nicht.

Durch diese Einschränkung zeigen Seifen eigenständige Charakteristika und sind als im Vergleich zu synthetischen Detergentien wie Laurylethersulfat oder SLS unabhängige Produktklasse zu betrachten.

Die von uns entwickelte Methode zur Bestimmung der Adsorption von Laurylethersulfat auf der Haut simuliert einen Waschvorgang, wie er z.B. beim täglichen Duschen auftritt:

Zunächst werden die Unterarminnenseiten mit Hilfe Isolierband in mehrere Testareale unterteilt. Anschließend wird das zu behandelnde Areal mit etwa 50 ml Leitungswasser (T = 38 ± 1 °C) angefeuchtet. Darauf folgt die Applikation des Laurylethersulfats. Während der Applikationszeit von 45 s verteilt der Proband die Laurylethersulfat-Lösung mit 2 Fingern durch gleichmäßige Kreisbewegungen auf dem Areal. Nach Ende der Applikationszeit wird das Testareal mit etwa 950 ml Leitungswasser (T = 38 ± 1°C) gespült und mit einem sauberen Zellstofftuch vorsichtig trocken getupft. Zur Desorption wird ein Kunststoffring mit einem Innendurchmesser von 25 mm schlüssig auf die Unterarminnenseite des Probanden gedrückt. Anschließend werden 1000 µl einer 1% Triton X-100 Lösung in den Ring eingefüllt. Für die Dauer von 1 min wird mit der Spitze eines runden, teflonbeschichteten Spatels gleichmäßig auf der Haut geschabt. Dies führt zu einer Suspendierung von Korneozyten. Zur Probenaufbereitung für die Analyse werden die Proben zentrifugiert und 800 µl des Überstands abgezogen. Der Überstand wird mittels lonenpaarchromatographie auf seinen Laurylethersulfat-Gehalt untersucht. Die Effektivität der beschriebenen Desorptions-Methode liegt bei 79%, d.h. 79% der sich tatsächlich auf der Haut befindenen Laurylethersulfat-Menge werden erfaßt.

Die Ergebnisse der Experimente zur Adsorption von Laurylethersulfat zeigen, daß Natriumlaurylethersulfat bei Waschvorgängen unter Anwendungsbedingungen an die Hautoberfläche adsorbiert/bindet.

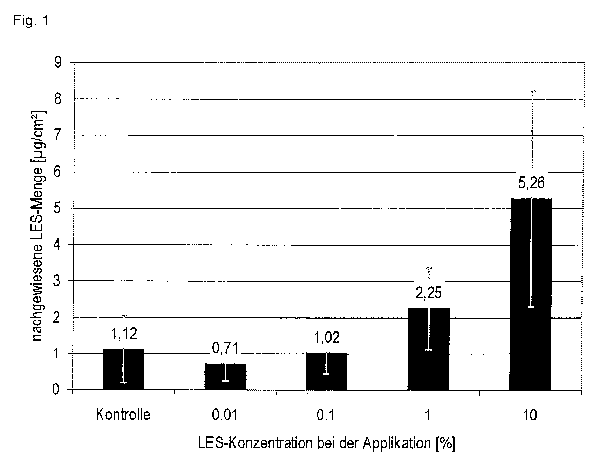

Erstaunlicherweise steigt die an der Hautoberfläche adsorbierte Tensidmenge unter Anwendungsbedingungen auch oberhalb der maximal erreichbaren Monomerenkonzentration (CMC) weiter an. Fig. 1 zeigt die Abhängigkeit der adsorbierten Menge an Laurylethersulfat von der Laurylethersulfat-Konzentration. Die CMC des verwendeten Laurylethersulfats liegt zwischen 0,01 und 0,1 Gew.-%.

Aufgabe der vorliegenden Erfindung war es daher, den aufgedeckten Übelständen abzuhelfen.

Erstaunlicherweise, und darin liegt die Lösung begründet, werden die Übelstände erfindungsgemäß beseitigt durch ein Verfahren zur Herstellung milder waschaktiver kosmetischer oder dermatologischer Zubereitungen, dadurch gekennzeichnet, daß mittels üblicher Verfahren die kritische Mizellbildungskonzentration (CMC¹) eines oder mehrerer waschaktiver Tenside (Tensid A) bestimmt wird, daß hernach ein oder mehrere Co-Tenside (Tensid B) gewählt werden, und daß durch Variation der relativen Konzentrationen von Tensiden und Co-Tensiden zueinander die jeweiligen Konzentrationen sowie das Konzentrationsverhältnis zueinander ermittelt wird, bei dem die kritische Mizellbildungskonzentration (CMC²) einen gewünschten Wert annimmt, der niedriger ist als CMC¹, und daß hernach die üblichen Bestandteile einer waschaktiven kosmetischen oder dermatologischen Zubereitung zusammen mit Tensid A und Tensid B in den jeweils ermittelten Konzentrationen zusammengegeben werden.

Eine weitere Ausführungsform der vorliegenden Erfindung betrifft ein Verfahren zur Verminderung der Bindung von Tensiden an die Hautoberfläche, dadurch gekennzeichnet, daß mittels üblicher Verfahren die kritische Mizellbildungskonzentration (CMC¹) eines oder mehrerer waschaktiver Tenside mit der Neigung, an die Hautoberfläche zu binden (Tensid A) bestimmt wird, daß hernach ein oder mehrere Co-Tenside (Tensid B) gewählt werden, und daß durch Variation der relativen Konzentrationen von Tensiden und Co-Tensiden zueinander die jeweiligen Konzentrationen sowie das Konzentrationsverhältnis zueinander ermittelt wird, bei dem die kritische Mizellbildungskonzentration (CMC²) einen gewünschten Wert annimmt, der niedriger ist als CMC¹, und daß hernach die üblichen Bestandteile einer waschaktiven kosmetischen oder dermatologischen Zubereitung zusammen mit Tensid A und Tensid B in den jeweils ermittelten Konzentrationen zusammengegeben werden.

Weiterhin wird die Erfindung verkörpert durch die Verwendung eines oder mehrerer Co-Tenside (Tensid B), die nicht dem Tensid Natriumlaurylethersulfat entsprechen im Gemisch mit dem Tensid Natriumlaurylethersulfat, zur Verminderung der Bindung von Tensid A an die Hautoberfläche.

Ferner betrifft die Erfindung die Verwendung eines oder mehrerer Co-Tenside (Tensid B) zur Herstellung milder waschaktiver kosmetischer oder dermatologischer Zubereitungen mit einem weiteren Gehalt an einem oder mehreren waschaktiven Tenside mit der Neigung, an die Hautoberfläche zu binden (Tensid A), bei deren Gebrauch eine Verminderung oder Verhinderung der Bindung von Tensid A an die Hautoberfläche erzielt wird.

Die vorliegende Erfindung betrifft in einer besonderen Ausführungsform waschaktive haarkosmetische Zubereitungen, landläufig als Shampoos bezeichnet. Insbesondere betrifft die vorliegende Erfindung haarkosmetische Wirkstoffkombinationen und Zubereitungen zur Pflege der Kopfhaut.

Überraschend und für den Fachmann nicht vorhersehbar stellte sich heraus, daß der Zusatz von Cotensiden zu Laurylethersulfat zu einer Verminderung der an die Haut adsorbierten Laurylethersulfat-Menge führt. Parallel durchgeführte Hautverträglichkeitsstudien belegen die durch den Zusatz von Cotensiden gesteigerte Milde der Produkte.

In Tabelle 1 wird die Reduzierung der Adsorption von Laurylethersulfat durch die Zugabe verschiedener Co-Tenside zu einer Laurylethersulfat-Lösung verdeutlicht.

**Tabelle 1**

| Cotensid | Konzentration des Cotensids | Effekt auf die adsorbierte Laurylethersulfat-Menge | Signifikanz |
|---|---|---|---|
| Natriumlauroylglutamat | 1,5% | Reduktion um 31% | Ja |
| Dinatriumlaurethsulfosuccinat | 3% | Reduktion um 28% | Ja |
| Natriumlauroylsarcosinat | 3% | Reduktion um 23% | Ja |
| Natriumcocoamphoacetat | 3% | Reduktion um 27% | Ja |
| Cocamidopropylbetain | 3% | Reduktion um 31% | Ja |
| Decylglukosid | 5% | Reduktion um 29% | Ja |
| Laurinsäure, pH9 | 1% | Reduktion um 15% | Ja |

Dieses Ergebnis ist umso erstaunlicher, als daß der Zusatz von einem oder mehreren Co-tensiden zu einer Erhöhung des Aktivgehalts an Tensiden in der Formulierung führt. Der Fachmann würde als Folge dieser Konzentrationserhöhung eine Reduzierung der Hautmilde erwarten.

Überraschenderweise stellte sich das gegenteilige Ergebnis ein: Die Erhöhung der Gesamttensidkonzentration führte zu einer Erhöhung der Milde der Produkte.

Noch eine weitere Ausführungsform der vorliegenden Erfindung betrifft die Verwendung eines oder mehrerer Co-Tenside (Tensid B) im Gemisch mit einem oder mehreren Tensiden die sich von Tensid B unterscheiden (Tensid A), zur Verminderung der Bindung von Tensid A an die Hautoberfläche.

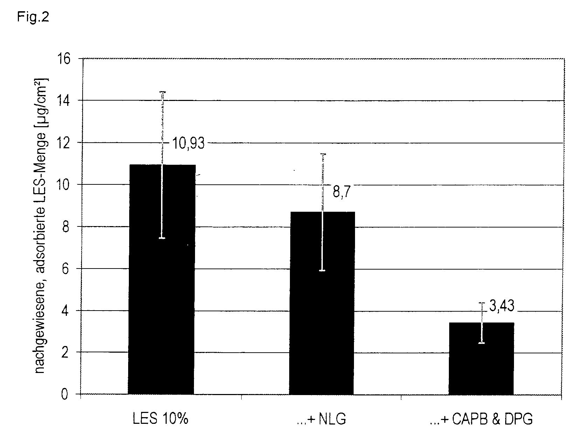

Abbildung 2 zeigt die Reduzierung der Laurylethersulfat-Adsorption durch Zugabe verschiedener Co-Tenside zu einer Laurylethersulfat-Lösung. LES bedeutet dabei Natriumlaurylethersulfat. NLG bedeutet Natriumlauroylglutamat. CAPB bedeutet Cocamidopropylbetain. DPG bedeutet Decylpolyglukosid.

Beispielsweise manifestiert sich die vorliegende Erfindung in waschaktiven kosmetischen oder dermatologischen Zubereitungen, enthaltend:
(a) mehr als 9,0 Gew.-% Laurylethersulfat,
(b) ein oder mehrere anionische Tenside, gewählt aus der Gruppe der N-Acylaminosäuren und deren Salze,
(c) anderer Tenside wie Cocamidopropyl Betaine, Decyl oder Dodecyl Polyglucoside, Disodium Laureth Sulfosuccinate, Sodium Lauroyl Sarcosinate, Trilaureth 4-Phosphate, Sodium Cocoamphoacetate, Disodium Cocoamphoacetate.
(c) weniger als 5,0 Gew.-% an anorganischen Salzen.

Weiterhin wird die vorliegende Erfindung verwirklicht durch waschaktive kosmetische oder dermatologischen Zubereitungen, enthaltend:
(a) mehr als 9,0 Gew.-% Laurylethersulfat,
(b) mehr als 1,0 Gew.-% , insbesondere mehr als 1,75 Gew.-% eines oder mehrerer anionischer Tenside, gewählt aus der Gruppe der N-Acylaminosäuren und deren Salze,
(c) weniger als 5,0 Gew.-% an anorganischen Salzen.

Tensid B kann vorteilhaft gewählt werden aus der Gruppe der N-Acylaminosäuren und deren Salze, wobei dieses Tensid oder diese Tenside in waschaktiven kosmetischen oder dermatologischen Zubereitungen in Konzentrationen vorliegt oder vorliegen, die größer als 3,0 Gew.-% betragen, bezogen auf das Gesamtgewicht der Zubereitungen, zur Verminderung des Aufziehens von Laurylethersulfat auf menschliche Haut während des Waschvorganges oder zur Entfernung von Laurylethersulfat von menschlicher Haut.

Es ist an sich bekannt, daß N-Acylaminosäuren und deren Salze milde Tenside mit brauchbarer Schaumwirkung und guter Waschwirkung darstellen (H. P. Fiedler, Lexikon der Hilfsstoffe für Pharmazie, Kosmetik und angrenzende Gebiete, 4.Auflage, S 108, Stichwort "N-Acylglutaminsäure")

In der Schrift "Surface Active N-Acylglutamate: Preparation of Long Chain N-Acylglutamic Acid" (M.Takehara, 1.Yoshimura, K.Takizawa, R. Yoshida; Journal of the American Oil Chemists' Society Vol.49, S.157 ff.) wird das JP-Patent 29 444 (1964) zitiert, demzufolge Acylglutamate lindernd auf von anderen anionischen Tensiden wie Natriumalkylbenzolsulfonaten und Natriumlaurylsulfat hervorgerufene Hautirritationen wirken solle.

Die DE-OS 43 04 066 beschreibt eine Zubereitung mit einem Gehalt an 12 Gew.-% Natriumlaurylethersulfat und 3 Gew.-% Natriumcocoylglutamat. Gegenstand dieser Schrift ist jedoch die Verwendung von Elektrolyten zur Verhinderung der Penetration der in den Reinigungsmitteln enthaltenen grenzflächenaktiven Substanzen und/oder anderer in diesen Reinigungsmitteln enthaltenen Substanzen in die äußeren Hautschichten - in der zuvor erwähnten Zubereitung sind dann auch 8 Gew.-% an Kochsalz zugegen, auf dessen Gegenwart der Fachmann die Verminderung des Reizpotentials des Natriumlaurylethersulfates zurückführt.

Vorteilhaft können die Acylaminosäuren (wobei im Rahmen der vorliegenden Offenbarung auch die Acylpeptide zu den Acylaminosäuren gerechnet werden) bzw. deren Salze gewählt werden aus der Gruppe
1. Acylglutamate, beispielsweise Natriumacylglutamate, Di-TEA-palmitoylaspartat und Natrium Caprylsäure/Caprinsäureglutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroylsarcosinat, Natriumlauroylsarcosinat und Natriumcocoylsarkosinat,
4 Taurate, beispielsweise Natriumlauroyltaurat und Natriummethylcocoyltaurat,
5. Acyllysinate, beispielsweise Laroyllysin
6. Acylalaninate
7. Acylglycinate

Weiterhin können die folgenden Co-Tenside eine vorteilhafte Wirkung besitzen:
1. Cocamidopropyl Betaine
2. Decyl Polyglucoside
3. Dodecyl Polyglucoside
4. Disodium Laureth Sulfosuccinate
5. Sodium Lauroyl Sarcosinate
6. Trilaureth 4-Phosphate
7. Sodium Cocoamphoacetate
8 Disodium Cocoamphoacetate.

Insbesondere vorteilhaft ist es im Sinne der vorliegenden Erfindung, als Acylaminosäure bzw. deren Salze Acylglutaminsäure bzw. Acylglutamate, insbesondere Natriumacylglutamate zu verwenden, die sich durch folgende Strukture auszeichnen:

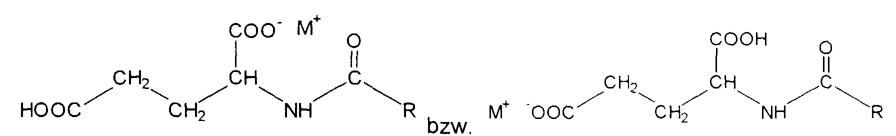

Von den Natriumacylglutamaten wiederum haben sich das Natriumcocoylglutamat, das Natriumlauroylglutamat, das Natriummyristoylglutamat, das Natriumstearoylglutamat und das Natriumtallowylglutamat als besonders vorteilhaft herausgestellt.

Die Zusammensetzungen enthalten gemäß der Erfindung außer den vorgenannten Tensiden gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die nachfolgenden Beispiele sollen die vorliegende Erfindung verdeutlichen, ohne sie einzuschränken Alle Mengenangaben, Anteile und Prozentanteile sind, soweit nicht anders angegeben, auf das Gewicht und die Gesamtmenge bzw auf das Gesamtgewicht der Zubereitungen bezogen.

| **Beispiel 1** | |
|---|---|
| | Gew.-% |
| Natrium Laurethsulfat (27,5 %-ige Lösung) | 48,00 |
| Cocoamidopropylbetain (33 %-ige Lösung) | 5,00 |
| Natriumcocoylglutamat (25 %-ige Lösung) | 5,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 2** | |
|---|---|
| | Gew.-% |
| Natrium Laurethsulfat (27,5 %-ige Lösung) | 40,00 |
| Cocoamidopropylbetain (33 %-ige Lösung) | 10,00 |
| Natriumcocoylglutamat (25 %-ige Lösung) | 8,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt

| **Beispiel 3** | |
|---|---|
| | Gew.-% |
| Natrium Laurethsulfat (27,5 %-ige Lösung) | 30,00 |
| Cocoamidopropylbetain (33 %-ige Lösung) | 15,00 |
| Natriumcocoylglutamat (25 %-ige Lösung) | 4,5,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 4** | |
|---|---|
| | Gew.-% |
| Natrium Laurethsulfat (27,5 %-ige Lösung) | 43,00 |
| Cocoamidopropylbetain (33 %-ige Lösung) | 11,00 |
| Natriumcocoylglutamat (25 %-ige Lösung) | 4,50 |
| Decylglucosid (50 %-ige Lösung) | 2,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s |
| Wasser | ad 100,00 |

Die Bestandteile werden bei Raumtemperatur zusammengegeben und homogen verrührt.

| **Beispiel 5** | |
|---|---|
| | Gew.-% |
| Natrium Laurethsulfat (27,5 %-ige Lösung) | 35,00 |
| Cocoamidopropylbetain (33 %-ige Lösung) | 8,00 |
| Natriumcocoylglutamat (25 %-ige Lösung) | 5,00 |
| Decylglucosid (50 %-ige Lösung) | 4,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 6** | |
|---|---|
| | Gew.-% |
| Natrium Laurethsulfat (27,5 %-ige Lösung) | 25,00 |
| Cocoamidopropylbetain (33 %-ige Lösung) | 14,00 |
| Natriumcocoylglutamat (25 %-ige Lösung) | 6,00 |
| Decylglucosid (50 %-ige Lösung) | 3,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q s. |
| Wasser | ad 100,00 |

Die Bestandteile werden bei Raumtemperatur zusammengegeben und homogen verrührt

| **Beispiel 7** | |
|---|---|
| | Gew.-% |
| Natrium Laurethsulfat (27,5 %-ige Lösung) | 47,00 |
| Natrium Cocoamphoacetat (36 %-ige Lösung) | 9,00 |
| Natriumcocoylglutamat (25 %-ige Lösung) | 6,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 8** | |
|---|---|
| | Gew.-% |
| Natrium Laurethsulfat (27,5 %-ige Lösung) | 41,00 |
| Natrium Cocoamphoacetat (36 %-ige Lösung) | 6,50 |
| Natriumcocoylglutamat (25 %-ige Lösung) | 7,50 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 9** | |
|---|---|
| | Gew.-% |
| Natrium Laurethsulfat (27,5 %-ige Lösung) | 41,00 |
| Natrium Cocoamphoacetat (36 %-ige Lösung) | 6,50 |
| Natriumlauroylglutamat (25 %-ige Lösung) | 5,50 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

| **Beispiel 10** | |
|---|---|
| | Gew.-% |
| Natrium Laurethsulfat (27,5 %-ige Lösung) | 32,00 |
| Natrium Cocoamphoacetat (36 %-ige Lösung) | 5,00 |
| Natriumcocoylglutamat (25 %-ige Lösung) | 5,00 |
| PEG-40 hydriertes Rizinusöl | 0,50 |
| PEG-100 hydriertes Glycerylpalmitat | 0,50 |
| Natriumbenzoat | 0,45 |
| Natriumsalicylat | 0,20 |
| Citronensäure | 0,50 |
| Parfum | q.s. |
| Wasser | ad 100,00 |

Die Bestandteile werden bei Raumtemperatur zusammengegeben und homogen verrührt.

## Patentansprüche

1. Verfahren zur Herstellung milder waschaktiver kosmetischer oder dermatologischer Zubereitungen, dadurch gekennzeichnet, daß mittels üblicher Verfahren die kritische Mizellbildungskonzentration (CMC¹) eines oder mehrerer waschaktiver Tenside (Tensid A) bestimmt wird, daß hernach ein oder mehrere Co-Tenside (Tensid B) gewählt werden, und daß durch Variation der relativen Konzentrationen von Tensiden und Co-Tensiden zueinander die jeweiligen Konzentrationen sowie das Konzentrationsverhältnis zueinander ermittelt wird, bei dem die kritische Mizellbildungskonzentration (CMC²) einen gewünschten Wert annimmt, der niedriger ist als CMC¹, und daß hernach die üblichen Bestandteile einer waschaktiven kosmetischen oder dermatologischen Zubereitung zusammen mit Tensid A und Tensid B in den jeweils ermittelten Konzentrationen zusammengegeben werden.

2. Verfahren zur Verminderung der Bindung von Tensiden an die Hautoberfläche, dadurch gekennzeichnet, daß mittels üblicher Verfahren die kritische Mizellbildungskonzentration (CMC¹) eines oder mehrerer waschaktiver Tenside mit der Neigung, an die Hautoberfläche zu binden (Tensid A) bestimmt wird, daß hernach ein oder mehrere Co-Tenside (Tensid B) gewählt werden, und daß durch Variation der relativen Konzentrationen von Tensiden und Co-Tensiden zueinander die jeweiligen Konzentrationen sowie das Konzentrationsverhältnis zueinander ermittelt wird, bei dem die kritische Mizellbildungskonzentration (CMC²) einen gewünschten Wert annimmt, der niedriger ist als CMC¹, und daß hernach die üblichen Bestandteile einer waschaktiven kosmetischen oder dermatologischen Zubereitung zusammen mit Tensid A und Tensid B in den jeweils ermittelten Konzentrationen zusammengegeben werden.

3. Verwendung eines oder mehrerer Co-Tenside (Tensid B) im Gemisch mit einem oder mehreren Tensiden die sich von Tensid B unterscheiden (Tensid A), zur Verminderung der Bindung von Tensid A an die Hautoberfläche.

4. Verwendung eines oder mehrerer Co-Tenside (Tensid B) zur Herstellung milder waschaktiver kosmetischer oder dermatologischer Zubereitungen mit einem weiteren Gehalt an einem oder mehreren waschaktiven Tenside mit der Neigung, an die Hautoberfläche zu binden (Tensid A), bei deren Gebrauch eine Verminderung oder Verhinderung der Bindung von Tensid A an die Hautoberfläche erzielt wird.

5. Verfahren oder Verwendung nach einem der vorhergehenden Ansprüche, dadurch gekennzeichnet, daß als Tensid A das Natriumlaurylethersulfat gewählt wird.

6. Verfahren oder Verwendung nach einem der vorhergehenden Ansprüche, dadurch gekennzeichnet, daß als Tensid B ein oder mehrere anionische Tenside aus der Gruppe der N-Acylaminosäuren und deren Salze gewählt wird.

7. Verfahren oder Verwendung nach Anspruch 6, dadurch gekennzeichnet, daß das Tensid B aus der folgenden Gruppe gewählt wird:
- der Acylglutamate, beispielsweise Natriumacylglutamate, Di-TEA-palmitoylaspartat und Natrium Caprylsäure/Caprinsäureglutamat,
- der Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- der Sarcosinate, beispielsweise Myristoyl Sarcosin, TEA-lauroylsarcosinat, Natriumlauroylsarcosinat und Natriumcocoylsarkosinat,
- der Taurate, beispielsweise Natriumlauroyltaurat und Natriummethylcocoyltaurat,
- der Acyllysinate, beispielsweise Laroyllysin
- der Acylalaninate
- der Acylglycinate
- der Betaine, beispielsweise Cocamidopropylbetaine
- der Amphoacetate, beispielsweise Natriumcocoamphoacetate oder Dinatriumcocoamphoacetate
- der Polyglukoside, beispielsweise Decyl oder Dodecylpolyglucoside
- der Sulfosuccinate, beispielsweise Dinatriumlauroylsulfosuccinat
- des Tensids Trilaureth-4-Phosphat
